**Europäisches Patentamt**

**European Patent Office**

**Office européen des brevets**

(11) Numéro de publication : **0 472 475 A2**

(12) # DEMANDE DE BREVET EUROPEEN

(21) Numéro de dépôt : **91420221.3**

(51) Int. Cl.⁵ : **A61F 2/38**

(22) Date de dépôt : **04.07.91**

(30) Priorité : **11.07.90 FR 9009191**

(43) Date de publication de la demande :
**26.02.92 Bulletin 92/09**

(84) Etats contractants désignés :
**AT BE CH DE DK ES FR GB GR IT LI LU NL SE**

(71) Demandeur : **Bousquet, Gilles**
**Chemin de Marandon**
**F-42000 Saint-Etienne (FR)**
(71) Demandeur : **Le Beguec, Pierre**
**11, Galerie du Théatre**
**F-35000 Rennes (FR)**
(71) Demandeur : **Rambert, André**
**Les Fontanelles 10 bis, rue du Docteur**
**Bonhomme**
**F-69003 Lyon (FR)**

(72) Inventeur : **Bousquet, Gilles**
**Chemin de Marandon**
**F-42000 Saint-Etienne (FR)**
Inventeur : **Le Beguec, Pierre**
**11, Galerie du Théatre**
**F-35000 Rennes (FR)**
Inventeur : **Rambert, André**
**Les Fontanelles 10 bis, rue du Docteur**
**Bonhomme**
**F-69003 Lyon (FR)**

(74) Mandataire : **Maureau, Philippe et al**
**Cabinet Germain & Maureau Le Britannia -**
**Tour C 20, bld Eugène Déruelle Boîte Postale**
**3011**
**F-69392 Lyon Cédex 03 (FR)**

(54) **Prothèse bicondylienne du genou.**

(57)     Cette prothèse est du type comportant deux éléments articulés l'un à l'autre par un axe d'articulation (4), à savoir un élément fémoral (3) reproduisant les condyles du fémur et un élément tibial reproduisant le plateau tibial, chacun de ces éléments (2,3) étant solidaire, par sa face opposée à celle tournée vers l'autre élément, d'une tige d'ancrage (22,34) destinée à être implantée, avec ou sans ciment, dans le canal médullaire de l'os correspondant.

Les moyens de liaison, au plateau tibial (21), du support (23) de l'axe d'articulation (4) sont constitués par une queue (25) orientée radialement à l'axe du palier (24) et engagée dans un puits (26) ménagé dans le plateau tibial.

La queue (25) et le puits (26) du plateau tibial destiné à la recevoir présentent une section transversale non circulaire et le plateau tibial (21) présente, sur sa face en contact avec les condyles (31), deux saignées (21e) de forme complémentaire de celle du secteur embrassé des condyles (31) et dont chacune est destinée à être engagée sur le secteur précité du condyle (31).

EP 0 472 475 A2

FIG.1

La présente invention concerne une prothèse bicondylienne du genou, c'est-à-dire une prothèse comportant un élément fémoral reproduisant les condyles du fémur et un élément tibial reproduisant le plateau tibial, chacun de ces éléments étant solidaire, par sa face opposée à celle tournée vers l'autre élément, d'une tige d'ancrage destinée à être implantée, avec ou sans ciment, dans le canal médullaire de l'os correspondant.

Il existe actuellement deux types de prothèses bicondyliennes. Dans les premières, dites à glissement, l'élément fémoral et l'élément tibial sont dissociés et, de ce fait, elles possèdent deux degrés de liberté, à savoir la rotation articulaire et les rotations interne et externe du tibia, mais elles ne possèdent aucun moyen de lutter contre les laxités antéro-postérieures et latérales.

Dans les secondes, dites à charnière, l'élément tibial est lié à l'élément fémoral par un axe d'articulation qui élimine un degré de liberté puisqu'il interdit les rotations interne et externe du tibia. En outre, la rotation du tibia autour d'un axe transversalement immobile ne permet pas de reproduire fidèlement l'articulation d'un genou sain.

Par le brevet français 81 13723, on connait une prothèse du second type dans laquelle, pour permettre au plateau tibial de suivre le profil des condyles sans perte de contact avec le plateau tibial, le palier de l'axe d'articulation, solidaire du plateau tibial, est constitué par une lumière débouchant vers le haut. En outre, pour faire bénéficier cette prothèse d'un second degré de liberté et permettre ainsi les rotations interne et externe du tibia, des jeux mécaniques importants sont prévus entre l'axe et son palier, d'une part et entre le palier de l'axe et les paliers de la chape fémorale, d'autre part.

Il en est résulté non seulement une grande laxité mais aussi une grande instabilité, très gênante pour la marche.

Par le brevet US 4 301 553, on connaît une prothèse du type précité, constituée de quatre éléments essentiels, à savoir un élément fémoral supportant une chape pour l'axe d'articulation de cette prothèse, les faces cylindriques des moyeux de cette chape étant sensés reproduire les condyles, un élément tibial comportant un plateau sur lequel est fixé le support du moyeu central de l'articulation, chacun de ces deux éléments étant pourvu d'une tige de fixation dans le canal médullaire de l'os correspondant et, enfin, deux plateaux intermédiaires engageables sur le support du moyeu central solidaire de l'élément tibial et dont celui supérieur présente deux surfaces concaves en forme de segments de cylindre et destinées à servir de sièges aux surfaces extérieures cylindriques des deux moyeux de la chape solidaire de l'élément fémoral.

Dans cette prothèse, la tige intramédullaire de l'élément tibial présente un puits conique et le plateau intermédiaire inférieur est pourvu d'une queue conique engageable dans le puits conique de la tige intramédullaire de l'élément tibial, cet engagement conservant à la tige du plateau intermédiaire inférieur une liberté de rotation et de déplacement axial pour procurer au patient une possibilité de rotation du tibia autour de son axe et une possibilité de flexion importante sans blocage prématuré.

Cette prothèse présente donc l'avantage, sur celles précitées, d'éliminer toute laxité-antéro-postérieure et latérale, tout en permettant une flexion importante du genou. En outre, la liberté de rotation du palier de l'axe d'articulation autour d'un axe sensiblement perpendiculaire au plateau tibial, c'est-à-dire sensiblement parallèle à l'axe de la tige intramédullaire de l'élément tibial, autorise les rotations interne et externe du tibia.

Cette prothèse présente le double inconvénient de ne pas limiter suffisamment la laxité latérale et d'utiliser essentiellement l'axe d'articulation et ses moyeux pour la transmission des efforts que l'on sait très importants entre les éléments tibial et fémoral de cette prothèse et qui provoquent une usure rapide de l'axe d'articulation et sa rupture prématurée qui impose au patient une nouvelle opération pour pose d'une nouvelle prothèse.

La présente invention vise à remédier à tous ces inconvénients. A cet effet, dans la prothèse qu'elle concerne et qui est du type à charnière, dont le palier de l'axe d'articulation des deux éléments est solidaire d'un support lié au plateau tibial par des moyens permettant, d'une part, sa rotation autour d'un axe sensiblement perpendiculaire à la face de ce plateau tournée vers l'élément fémoral et, d'autre part, le déplacement transversal de cet axe d'articulation dans une direction parallèle au second axe précité c'est-à-dire dans une direction sensiblement perpendiculaire au plateau tibial, les moyens de liaison, au plateau tibial, du support de l'axe d'articulation étant constitués par une queue orientée radialement à l'axe du palier et engagée dans un puits, de section transversale complémentaire de celle de la queue précitée, ménagé, pour la recevoir avec possibilité de coulissement axial, dans le plateau tibial, d'une part, la queue précitée et le puits du plateau tibial destiné à la recevoir présentent une section transversale non circulaire et, d'autre part, le plateau tibial présente, sur sa face en contact avec les condyles, deux saignées de forme complémentaire de celle du secteur embrassé des condyles et dont chacune est destinée à être engagée sur le secteur précité du condyle.

Ces dispositions présentent non seulement l'avantage de réduire considérablement les risques de laxité latérale en assurant un meilleur guidage du plateau tibial sur les condyles du fémur, mais, surtout, celui de supprimer presque totalement les contraintes mécaniques entre l'axe d'articulation et ses moyeux et de conférer à cette prothèse une très grande longévité.

Comme on le sait, les courbures des condyles et de la trochlée d'un genou naturel n'ont pas le même rayon et le changement de courbure qui en résulte limite normalement le secteur de contact entre la plateau tibial et les condyles.

Cette limitation qui est pratiquement sans importance dans un genou sain, équipé de ligaments en bon état présente, évidemment, l'inconvénient de nuire à la stabilité dans un genou équipé d'une prothèse et dont les ligaments sont au moins distendus sinon totalement détériorés.

C'est pourquoi, suivant une forme d'exécution préférée de l'invention, d'une part, chaque saignée du plateau tibial présente, à son extrémité correspondant à l'avant du genou, une nervure de section transversale arrondie et dont au moins une partie de l'arête du sommet constitue un arc de cercle de même rayon et de même centre que ceux du fond de la partie postérieure de cette saignée et, d'autre part, chaque condyle de l'élément fémoral présente, dans le segment recouvert par la nervure précitée de la saignée correspondante du plateau tibial, lorsque la jambe du patient est en extension, un creux de forme complémentaire apte à loger cette nervure dans la position précitée de la jambe du patient.

Grâce à cette disposition, le contact entre chaque condyle et le plateau tibial est réalisé sur un secteur d'arc de cercle bien plus important qu'en l'absence de ces dispositions, ce qui améliore considérablement la stabilité du genou.

En outre, l'engagement des nervures des saignées du plateau tibial dans les creux des condyles sert aussi de butée limitant le déploiement de la jambe.

Enfin, l'augmentation de l'arc de cercle du secteur de contact entre les condyles de l'élément fémoral et les saignées du plateau tibial diminue les pressions unitaires, lors des transmissions d'efforts et, par conséquent, l'usure de ces éléments.

Il faut aussi noter que l'engagement des condyles de l'élément fémoral dans les saignées du plateau tibial participe, avec la section non circulaire de la queue du support du moyeu de l'axe d'articulation lié au moyeu tibial, à la liaison en rotation du plateau tibial et de l'élément fémoral lors des rotations latérales gauche et droite du tibia, ce qui assure une meilleure répartition des efforts et diminue encore les causes d'une usure locale.

De préférence, le plateau intermédiaire et le support du palier de l'axe d'articulation sont en un métal inaltérable et biocompatible tel qu'en alliage de titane éventuellement traité en surface pour recevoir une implantation ionique tandis que le plateau tibial est en une matière synthétique bio-compatible et présentant un faible coefficient de frottement par rapport au métal constitutif des pièces précitées. Une matière synthétique convenant parfaitement à cet usage est le polyéthylène haute densité, éventuellement traité en surface pour recevoir une implantation ionique.

Suivant une caractéristique simple de l'invention, les moyens de montage rotatif et de guidage du plateau tibial sur le plateau intermédiaire comprennent, d'une part, en saillie sur la face du plateau tibial tournée vers le plateau intermédiaire, un manchon cylindrique entourant son puits destiné à recevoir la queue du support de l'axe d'articulation et destiné à être engagé dans un trou traversant, prévu dans le plateau intermédiaire pour lui servir de palier et, d'autre part, en saillie sur la face du plateau intermédiaire tournée vers le plateau tibial, une virole cylindrique coaxiale au trou traversant du plateau intermédiaire et destinée à être engagée dans une gorge annulaire ménagée, pour la recevoir, dans la face inférieure du plateau tibial, autour du manchon cylindrique précité.

Suivant encore une autre caractéristique de cette prothèse ayant pour but de limiter les rotations interne et externe du tibia, le plateau tibial porte, sur sa face inférieure, c'est-à-dire sur sa face tournée vers le plateau intermédiaire, au moins une saillie en forme d'ergot et le plateau intermédiaire présente, sur sa face supérieure en regard avec le plateau tibial et en correspondance de chaque saillie précitée, une mortaise en arc de cercle, apte à recevoir cette saillie et à limiter la course angulaire du plateau tibial par ses extrémités dont chacune sert de butée à la saillie du plateau tibial.

De toute façon l'invention sera bien comprise à l'aide de la description qui suit en référence au dessin schématique annexé représentant, à titre d'exemple non limitatif, une forme d'exécution de cette prothèse :

Figure 1 est une vue en perspective montrant les deux éléments de cette prothèse articulés l'un à l'autre ;

Figure 2 est une vue de côté en élévation de la prothèse de figure 1 ;

Figure 3 est une vue en perspective éclatée de l'élément tibial de la prothèse des figures 1 et 2 ;

Figure 4 est une vue en perspective, par-dessous, du plateau tibial seul de l'élément tibial de figure 3 ;

Figure 5 est une vue en perspective éclatée de l'élément fémoral des figures 1 et 2.

Figure 6 est une vue partielle, en coupe suivant VI-VI de figure 1 montrant un agencement particulier des saignées et des condyles, la jambe du patient étant en extension ;

Figure 7 est une vue similaire à figure 6 correspondant aux positions de la jambe du patient, respectivement totalement et partiellement fléchie ;

Figure 8 est une vue en coupe VIII-VIII du figure 6.

Comme le montre le dessin, cette prothèse bicondylienne du genou est du type comportant deux éléments, à savoir un élément tibial 2 et un élément fémoral 3. L'élément tibial 2 est destiné à remplacer le plateau tibial endommagé d'un tibia. A cet effet, cet élément tibial 2 comporte un plateau tibial 21 solidaire, par exemple par assemblage et vissage, d'une tige d'ancrage 22 destiné à être implantée, avec ou sans ciment, dans le canal médullaire du tibia préalablement réséqué.

L'élément fémoral 3 comprend, essentiellement, deux nervures parallèles en forme de condyles 31 se prolongeant, vers l'avant, par un bouclier trochléen 32 destiné à recevoir la rotule anatomique ou une rotule prothétique. Entre les deux condyles 31, l'élément fémoral porte, sur sa face supérieure, c'est-à-dire celle tournée à l'opposé du plateau tibial 21 de l'élément tibial 2, un bossage évidé 33, destiné à recevoir la tige d'ancrage 34 devant servir à la fixation de cet élément fémoral 3 au fémur préalablement réséqué du patient. Cette tige d'ancrage 34 est destinée à être implantée, avec ou sans ciment, dans le canal médulaire du fémur du patient. L'axe de la tige 34 et, par conséquent, du bossage évidé 33, est incliné d'environ 4° par rapport au plan vertical médian de l'élément fémoral 3, ce qui permet de restituer à cette articulation le valgus physiologique.

Comme indiqué précédemment, la prothèse selon l'invention est du type comportant un axe d'articulation 4.

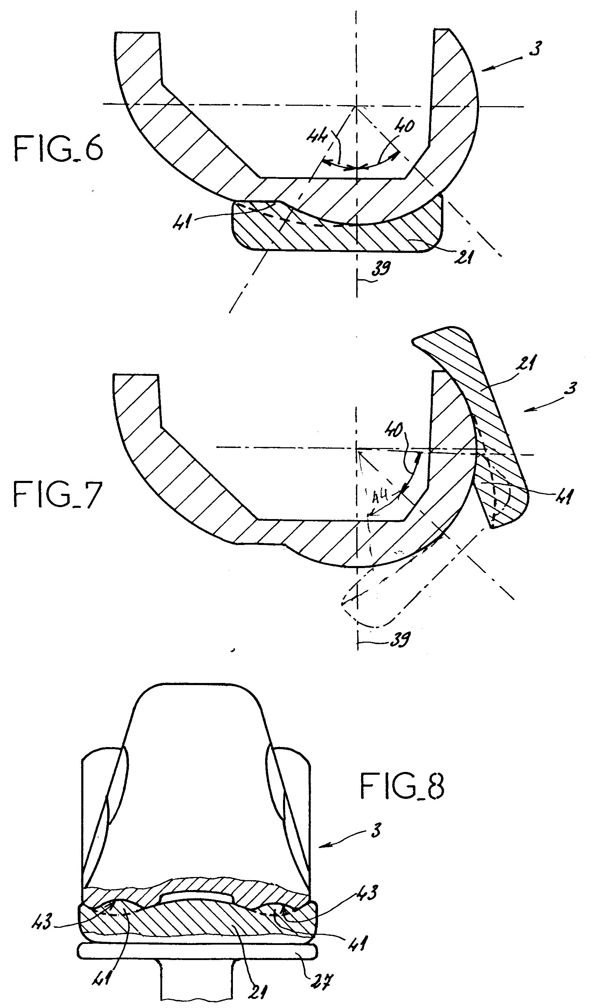

Pour permettre sa liaison à l'axe d'articulation 4, l'élément fémoral 3 présente deux paliers coaxiaux 35 (dont un est visible sur la figure 5) dont chacun est aménagé dans une pièce intermédiaire 36 destinée à servir d'entretoise, comme cela sera expliqué ci-après, après avoir été engagée, partiellement, dans un trou 37 aménagé, pour la recevoir, dans une nervure 38 prévue sur la face postérieure de chaque condyle 31 auquel elle sert de raidisseur. L'axe 4 se trouve donc lié rigidement à la pièce fémorale 3.

La liaison de l'axe d'articulation 4 à l'élément tibial est réalisée par l'intermédiaire d'un support 23 dans lequel est aménagé un palier 24 pour l'arbre 4 et qui porte une queue radiale 25 destinée à être engagée, avec possibilité de coulissement axial, dans un puits 26 de même section transversale qu'elle, aménagé, pour la recevoir et la guider, dans le plateau tibial 21.

La liaison ainsi réalisée entre l'axe d'articulation 4 et l'élément tibial 2 autorise donc des déplacements transversaux de l'axe 4 parallèlement à l'axe commun 5 de la queue 25 et du puits 26.

L'articulation de l'élément tibial 2 à l'élément fémoral 3 procure donc à cette prothèse un premier degré de liberté puisqu'autorisant un pivotement autour de l'axe 4.

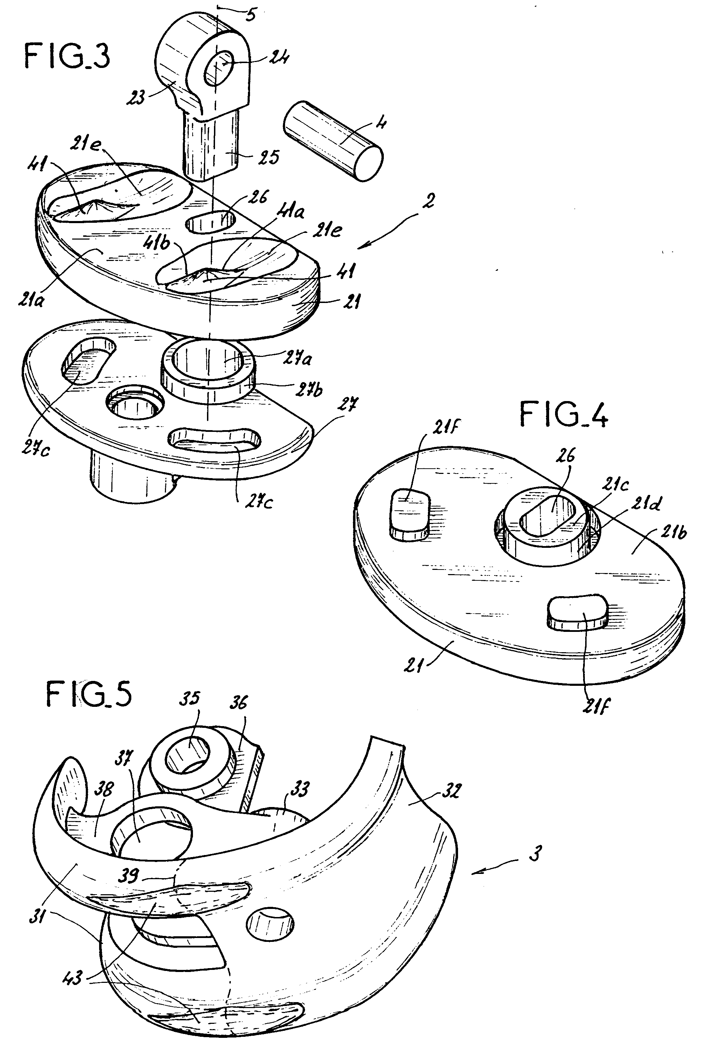

Il est possible de procurer à cette prothèse un second degré de liberté en autorisant une rotation de l'élément tibial 2, par rapport à l'élément fémoral 3, autour de l'axe 5 commun à la queue 25 et au puits 26. A cet effet, dans le but de diminuer les causes d'usure du plateau tibial 21 par les condyles 31 de l'élément fémoral 3, le plateau tibial 21 est monté pivotant, par rapport à la tige d'ancrage 22 de l'élément fémoral 2, autour de l'axe commun 5 de la queue 25 et du puits 26 destiné à la recevoir et il est prévu, lié rigidement à la tige d'ancrage 22, un plateau intermédiaire 27 sur lequel le plateau tibial 21 est monté avec possibilité de rotation autour de l'axe 5. A cet effet, d'une part, le plateau tibial 21 porte, en saillie sur sa face inférieure 21b, un manchon cylindrique 21c entourant son puits destiné à recevoir la queue 25 du support 24 de l'axe d'articulation 4, ce manchon étant destiné à être engagé dans un trou traversant 27a ménagé dans le plateau intermédiaire 27 pour lui servir de palier et, d'autre part le plateau intermédiaire 27 porte, sur sa face supérieure tournée vers le plateau tibial 21, une virole cylindrique 27b coaxiale au trou traversant 27a et destinée à être engagée dans une gorge annulaire 21d ménagée, pour la recevoir, autour du manchon cylindrique 21c du plateau tibial 21. Dans ce cas, la section transversale de la queue 25 et du puits 26 qui n'a plus à être de section circulaire présente, avantageusement, une section oblongue comme illustré sur la figure 3.

Pour limiter l'angle de pivotement du plateau tibial 21 sur le plateau intermédiaire 27 autour de l'axe 5, dans l'exemple illustré sur le dessin, le plateau intermédiaire 27 porte, sur sa face supérieure tournée vers le plateau tibial 21, deux mortaises 27c en forme d'arcs de cercle centrés sur l'axe 5 commun à la queue 25, au puits 26 et au trou traversant 27a tandis que le plateau tibial 21 porte, en saillie sur sa face inférieure 21b, et en correspondance de chacune des mortaises 27c, deux ergots 21f, également en forme d'arcs de cercle, mais représentant un secteur inférieur à celui des mortaises 27c, de manière à permettre un certain débattement angulaire du plateau tibial 21 par rapport au plateau intermédiaire 27. Ce manchon permet donc de procurer, sans aucune difficulté, à cette prothèse, les possibilités de rotations interne et externe du tibia.

Cette prothèse présente donc bien deux degrés de liberté puisque l'axe 4 autorise un premier degré de liberté tandis que le montage pivotant du plateau tibial 21 autorise le second.

En outre, le montage de l'axe 4 dans son support 24 mobile parallèlement à l'axe 5 permet de réaliser des condyles 31 de profil non circulaire, c'est-à-dire reproduisant, au plus près, le profil des condyles naturels d'un fémur.

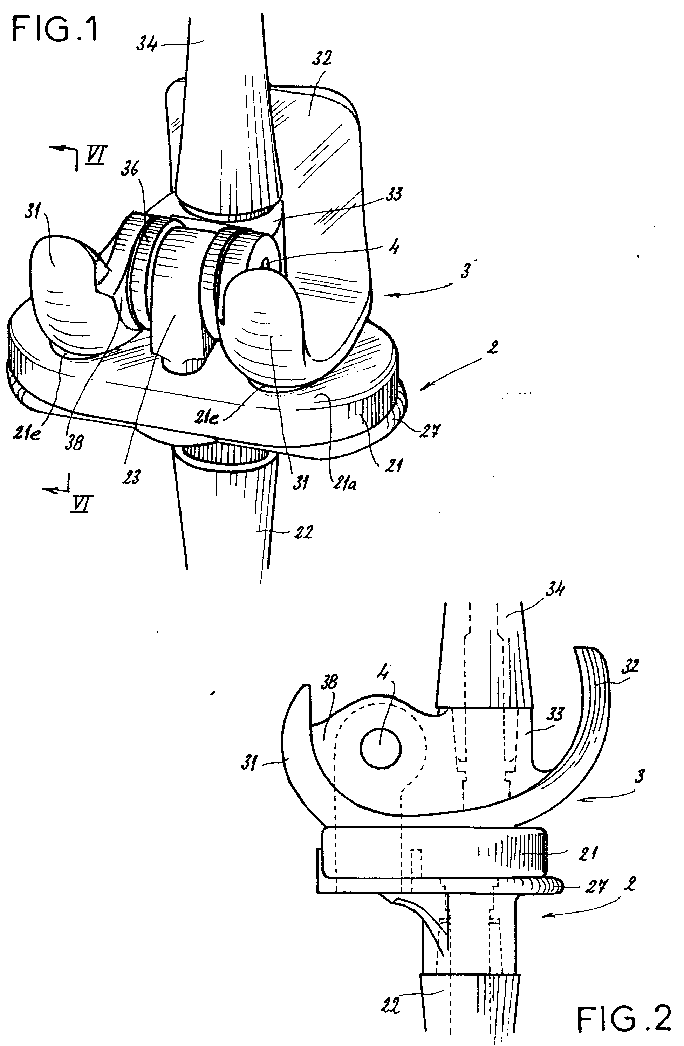

L'examen de la figure 1 montre, en outre, que la présence des pièces intermédiaires 36 dont chacune est disposée entre le support 23 de l'axe d'articulation 4 et l'une des nervures 38, a pour effet d'éliminer toute laxité latérale de cette prothèse. Par ailleurs, l'engagement de la queue 25 dans le puits 26 du plateau tibial 21 lui-même lié axialement au plateau intermédiaire 27 élimine toute laxité antéro-postérieure.

Pour améliorer encore la stabilité de cette prothèse, le plateau tibial 21 présente, sur sa face 21a en contact avec les condyles 31, deux saignées 21e dont chacune est destinée à loger partiellement et guider l'un des condyles 31 de l'élément fémoral 3.

Comme le montre la figure 6, les nervures reproduisant les condyles 31 et leur prolongément reproduisant les bords de la trochlée 32 de la pièce fémorale 3 n'ont pas le même rayon. Les segments de ces deux zones se rencontrent d'ailleurs en un plan transversal 39 visible sur les figures 6, 7. En conséquence, sans disposition particulière telle que celle décrite ci-après, l'arc de cercle du secteur de contact entre le plateau tibial 21 et le condyle 31 associé se réduit à l'arc de cercle 40 dès que la jambe du patient fléchit un peu puisque dans la zone bordant la trochlée 32, chaque nervure est d'un rayon supérieur à celui de sa zone reproduisant les condyles 31 et qu'il en est de même pour chaque saignée 21e du plateau tibial 21.

L'examen de la figure 7 où deux positions sont représentées, l'une intermédiaire en traits mixtes et l'autre en flexion maximale en traits pleins, montre bien la perte importante de contact entre la plateau tibial 21 et chaque condyle 31.

Pour cette raison, il est prévu une forme d'exécution perfectionnée de cette prothèse visant à remédier à ces inconvénients. Suivant cette forme d'exécution, comme le montrent les figures 3, 5 et 6 à 8, chaque saignée 21e du plateau tibial 21 présente, à son extrémité correspondant à l'avant du genou, c'est-à-dire à son extrémité située à droite sur la figure 3, une nervure 41 de section transersale arrondie et chaque condyle ou nervure 31 reconstituant un condyle présente, disposée à cheval sur la zone de raccordement 42 entre l'arc de cercle reproduisant le condyle et la courbure bordant la trochlée 32, une saignée 43 de forme complémentaire de celle de la nervure 41 précitée:

En outre, comme cela est plus particulièrement visible sur les figures 3 et 6 et 7, l'arête du sommet de chaque nervure 41 constitue, sur un premier tronçon 41a prenant naissance à l'opposé de l'extrémité avant de la saignée 21e, un arc de cercle de même rayon et de même centre que ceux du fond de la partie postérieure de la saignée 21 dans le prolongement de laquelle prend naissance cette nervure 41 tandis que le prolongement vers l'avant du genou, c'est-à-dire vers l'extrémité avant de la saignée 21 de chaque nervure 41, est rectiligne, c'est-à-dire constitué par une droite 41b.

Chaque creux 43 aménagé dans chaque condyle 31 occupe une position intérieure telle que la nervure 41 de la saignée 21e associée à ce condyle 31 se trouve engagée dans ce creux 43 lorsque la jambe du patient est en extension comme illustrée sur la figure 6.

Comme cela ressort à l'évidence de l'examen de la figure 6, dans cette position, la stabilité de l'articulation se trouve considérablement améliorée puisque non seulement par l'engagement de chaque nervure 41 dans le creux 43 correspondant mais, en outre, par le fait que les secteurs de contact entre les condyles 31 du plateau tibial 21 et l'élément fémoral 3, sont prolongés sur un arc surpérieur à celui 40 qui existait en l'absence de la nervure 41 et du creux 43.

Par ailleurs, à l'examen de la figure 7, on voit que, dans les autres positions de la jambe du patient, comme lorsqu'elle est en extension, la présence de la nervure 41 de chaque saignée 21e du plateau tibial 21 a pour effet d'augmenter l'arc de cercle du secteur de contact entre le plateau tibial 21 et chaque condyle 31 d'une longueur égale à l'arc de cercle 44 qui s'ajoute donc à l'arc de cercle précité 40. Cette augmentation du contact entre le plateau tibial 21 et l'élément fémoral 3 améliore donc considérablement la stabilité de cette articulation.

Naturellement, dans la position de flexion maximale représentée en traits pleins sur la figure 7, une partie de l'arc de cercle de contact 40 entre la plateau tibial 21 et l'élément fémoral 3 est perdue, ce qui rend d'autant plus intéressante la présence de la nervure 41.

Il faut noter enfin que, lorsque la jambe du patient est en extension et que le plateau tibial 21 occupe la position illustrée sur la figure 6, la nervure 41 de chaque saignée 21e est totalement engagée dans le creux 43 correpondant du condyle 31 associé, de sorte que le tronçon rectiligne 41b de chaque nervure 41 se trouve en contact avec le tronçon correspondant et de forme complémentaire du creux 43 associé , ce qui engendre un effet de butée limitant à la valeur désirée la position en extension de la jambe du patient.

Pour conférer à cette prothèse une douceur de fonctionnement la rendant plus confortable, sans nuire à sa longévité, le plateau intermédiaire 21 est réalisé en polyéthylène haute densité tandis que toutes les autres pièces de cette prothèse c'est-à-dire le plateau intermédiaire 27, l'axe d'articulation 4, son support 24 et la queue 25 de ce dernier, ainsi que l'ensemble de l'élément fémoral 3, sont réalisés en alliage de titane tel que celui connu dans le commerce sous la référence TA6V.

En outre, pour améliorer le glissement des surfaces frottantes, ces dernières qu'elles soient en polyéthylène haute densité ou en alliage de titane, ont avantageusement subi une implantation ionique. Toutes ces matières utilisées sont naturellement inaltérables et biocompatibles.

**Revendications**

1. Prothèse bicondylienne du genou, du type à charnière comportant deux éléments articulés l'un à l'autre par un axe d'articulation (4), à savoir un élément fémoral (3) reproduisant les condyles du fémur et un élément tibial reproduisant le plateau tibial, chacun de ces éléments (2,3) étant solidaire, par sa face opposée à celle tournée vers l'autre élément, d'une tige d'ancrage (22,34) destinée à être implantée, avec ou sans ciment, dans le canal médulaire de l'os correspondant, l'axe d'articulation (4) traversant des paliers (35) solidaires de l'élément fémoral (3) et un palier (24) associé à l'élément tibial (2), et du type dans lequel le palier (24), associé à l'élément tibial (2) est solidaire d'un support (23), lié au plateau tibial (21) par des moyens permettant, d'une part, sa rotation autour d'un axe (5) sensiblement perpendiculaire à la face (21a) de ce plateau (21) tournée vers l'élément fémoral (3) et, d'autre part, le déplacement transversal de cet axe d'articulation (4) dans une direction parallèle au second axe (5) précité, c'est-à-dire dans une direction sensiblement perpendiculaire au plateau tibial (21), les moyens de liaison, au plateau tibial (21), du support (23) de l'axe d'articulation (4) étant constitués par une queue (25) orientée radialement à l'axe du palier (24) et engagée dans un puits (26), de section transversale complémentaire de celle de la queue (25), ménagé, pour la recevoir avec possibilité de coulissement axial, dans le plateau tibial (21), et dans lequel le plateau tibial (21) est monté sur un plateau intermédiaire (27) avec possibilité de rotation autour de l'axe (5) du puits (26) destiné à recevoir la queue (25) du support (23) du palier (24) de l'axe d'articulation (4), caractérisée en ce que, d'une part, la queue (25) et le puits (26) du plateau tibial destiné à la recevoir présentent une section transversale non circulaire et d'autre part le plateau tibial (21) présente, sur sa face en contact avec les condyles (31), deux saignées (21e) de forme complémentaire de celle du secteur embrassé des condyles (31) et dont chacune est destinée à être engagée sur le secteur précité du condyle (31).

2. Prothèse selon la revendication 1, caractérisée en ce que, d'une part, chaque saignée (21e) du plateau tibial (21) présente, à son extrémité correspondant à l'avant du genou, une nervure (41) de section transversale arrondie et dont au moins une partie de l'arête du sommet constitue un arc de cercle de même rayon et de même centre que ceux du fond de la partie postérieure de cette saignée (21e) et, d'autre part, chaque condyle (31) de l'élément fémoral (3) présente, dans le segment recouvert par la nervure précitée de la saignée correspondante du plateau tibial (21), lorsque la jambe du patient est en extension, un creux (43) de forme complémentaire apte à loger cette nervure (41) dans la position précitée de la jambe du patient.

3. Prothèse selon l'une des revendications 1 ou 2, caractérisée en ce que les moyens de montage rotatif et de guidage du plateau tibial (21) sur le plateau intermédiaire (27) comprennent, d'une part, en saillie sur la face (21b) du plateau tibial (21) tournée vers le plateau intermédiaire (27), un manchon cylindrique (21c) entourant son puits (26) destiné à recevoir la queue (25) du support (23) de l'axe d'articulation (4) et destiné à être engagé dans un trou traversant (27a), prévu dans le plateau intermédiaire (27), pour lui servir de palier et, d'autre part, en saillie sur la face du plateau intermédiaire (27) tournée vers le plateau tibial (21), une virole cylindrique (27b) coaxiale au trou traversant (27a) du plateau intermédiaire (27) et destinée à être engagée dans une gorge annulaire (21d) ménagée, pour la recevoir, dans la face inférieure (21b) du plateau tibial (21), autour du manchon cylindrique (21c).

4. Prothèse selon l'une quelconque des revendications 1 à 3, caractérisée en ce que le plateau tibial (21) porte, sur sa face inférieure (21b), c'est-à-dire sur sa face tournée vers le plateau intermédiaire (27), au moins une saillie (21f) en forme d'ergot et le plateau intermédiaire (27) présente, sur sa face supérieure en regard avec le plateau tibial (21) et en correspondance de chaque saillie (21f), une mortaise (27c) en arc de cercle apte à recevoir cette saillie (21f) et à limiter la course angulaire du plateau tibial (21) par ses extrémités dont chacune sert de butée à la saillie (21f) du plateau tibial (21).

5. Prothèse selon l'une quelconque des revendications 1 à 4, caractérisée en ce que le plateau intermédiaire (27) et le support (23) du palier (24) de l'axe d'articulation (4) sont en un métal inaltérable et biocompatible tel qu'en alliage de titane éventuellement traité en surface pour recevoir une implantation ionique tandis que le plateau tibial (21) est en une matière synthétique biocompatible et présentant un faible coefficient de frottement par rapport au métal constitutif des pièces précitées telles que le polyéthylène haute densité, éventuellement traité en surface pour recevoir une implantation ionique.

FIG.1

FIG.2

EP 0 472 475 A2

FIG.3

FIG.4

FIG.5

9

FIG_6

FIG_7

FIG_8